# EUROPÄISCHE PATENTSCHRIFT

(11) **EP 3 755 398 B1**
(45) Veröffentlichungstag und Bekanntmachung des Hinweises auf die Patenterteilung: **29.11.2023**
(21) Anmeldenummer: 19709374.3
(22) Anmeldetag: 14.02.2019
(51) Int. Cl.: A61M 1/28, A61M 1/00

(54) **PERITONEALDIALYSEGERÄT**
PERITONEAL DIALYSIS DEVICE
APPAREIL DE DIALYSE PÉRITONÉALE

(30) Priorität: 21.02.2018 DE 102018103918
(43) Veröffentlichungstag der Anmeldung: 30.12.2020
(73) Patentinhaber: Fresenius Medical Care Deutschland GmbH, 61352 Bad Homburg (DE)
(72) Erfinder: WABEL, Peter, 61191 Rosbach (DE); WOLF, Klaus, 97450 Muedesheim (DE); WEISS, Stefan, 61352 Bad Homburg (DE)
(74) Vertreter: Herrmann, Uwe
(86) Internationale Anmeldenummer: PCT/EP2019/053685
(87) Internationale Veröffentlichungsnummer: WO 2019/162188

(56) Entgegenhaltungen:
- CN-U- 207 152 824
- DE-U1- 8 714 464
- US-A- 3 709 222

## Beschreibung

Die vorliegende Erfindung betrifft ein Peritonealdialysegerät mit Mitteln zur Durchführung einer Peritonealdialysebehandlung, wobei das Gerät eine Ablaufleitung aufweist, die mit dem Patientenkatheter unmittelbar oder mittelbar in Verbindung steht oder verbindbar ist und durch die gebrauchte Dialyselösung in einen Ablauf geleitet wird.

Im Bereich der Peritonealdialyse ist es üblich, dem Patienten Lösungsbeutel zur Verfügung zu stellen, die mit einer für den Patienten geeigneten Dialyselösung befüllt sind. Diese Lösungsbeutel schließt der Patient dann selbsttätig oder mit Unterstützung von medizinischem Fachpersonal an einen Einlaufschlauch, d.h. an den Patientenkatheter an, um das Peritoneum mit der frischen Dialyselösung zu füllen. Nach Abschluss der Behandlung, d.h. nach einer bestimmten Einwirkzeit wird die Lösung aus dem Bauchraum des Patienten wieder entfernt und in einen Drainbehälter oder Drainbeutel, d.h. in einen Behälter oder Beutel zur Aufnahme der gebrauchten Dialyselösung abgelassen.

DE 87 14 464 U1 offenbart einen sterilisierbaren bzw. sterilisierten Schlauchsatz aus Kunststoff mit Flüssigkeitsbeuteln und Verbindungskonnektoren an den Schlauchenden zur Durchführung von geräteunterstützen Flüssigkeitsübertragungen für die Peritonealdialyse. US 3 709 222 A betrifft ein Peritonealdialysegerät mit Ablaufleitungen, die mit dem Patientenkatheter verbindbar ist.

Der vorliegenden Erfindung liegt die Aufgabe zugrunde, ein Peritonealdialysegerät der eingangs genannten Art dahingehend weiterzubilden, dass auf Drainbehälter oder Drainbeutel verzichtet werden kann, wobei sichergestellt ist, dass nach der Behandlung stets eine zuverlässige und vollständige Entleerung der Ablaufleitung erreicht wird.

Diese Aufgabe wird durch ein Peritonealdialysegerät mit den Merkmalen des Anspruchs 1 gelöst. Danach ist vorgesehen, dass die Ablaufleitung nicht in einen Drainbehälter oder Drainbeutel mündet und dass die Ablaufleitung an einer Einmündung mit einer Belüftungsleitung verbunden ist, mittels derer Luft in die Ablaufleitung einleitbar ist, wobei in der Ablaufleitung und/oder in der Belüftungsleitung stromaufwärts der Einmündung ein Sterilfilter angeordnet ist und wobei in der Ablaufleitung stromaufwärts der Einmündung ein Absperrmittel vorgesehen ist, mittels dessen die Ablaufleitung zeitweise absperrbar ist.

Die Begriffe "stromabwärts" und "stromaufwärts" beziehen sich auf die Strömungsrichtung des zum Ablauf abfließenden Dialysats.

Durch die in die Ablaufleitung mündende Belüftungsleitung ist es möglich, die Ablaufleitung vollständig zu entleeren. Insbesondere bei der CAPD herrschen häufig undefinierte Druckverhältnisse, die von der Höhenlage des Patienten relativ zum Ablauf abhängen, so dass der ungünstige Fall eintreten kann, dass beim Entleeren des Patienten das in der Ablaufleitung befindliche Dialysat nicht vollständig abläuft, sondern in dieser stehen bleibt oder sogar unbemerkt zum Patienten zurückläuft, was mit Kontaminationen bzw. Infektionen einhergehen kann, die selbstverständlich unerwünscht sind.

Ferner besteht die Möglichkeit, dass die Ablaufleitung erhebliche Höhenunterschiede überbrücken muss und eine in der Ablaufleitung stehende Fluidsäule einen Unterdruck an dem Anschluss des Patienten und somit dem Bauchraum des Patienten verursachen kann. Dieser unerwünschte Effekt soll ebenfalls verhindert werden.

Erfindungsgemäß beträgt die Länge der Ablaufleitung mindestens 2 m. Dies ist eine Länge, die bei einem Höhenunterschied einen erheblichen Unterdruck im Peritoneum des Patienten verursacht.

Durch die erfindungsgemäße Belüftungsleitung wird dies verhindert, da die einströmende Luft den Ablauf des in der Ablaufleitung stehenden Restdialysats in den Abfluss bewirkt. Das Kontaminationsrisiko des Patienten sowie Risiken und die Beeinträchtigung des Wohlbefindens durch unerwünschte Druckverhältnisse ist dadurch wesentlich gemindert.

An dieser Stelle wird darauf hingewiesen, dass die vorliegende Erfindung nicht auf eine Behandlungsart, wie CAPD oder APD beschränkt ist, sondern beliebige Peritonealdialysebehandlungen bzw. dazu bestimmte Geräte und Verfahren umfasst. Die vorliegende Erfindung findet jedoch vorwiegend in der CAPD Anwendung. Vorzugsweise handelt es sich bei dem Peritonealdialysegerät somit um ein CAPD-Set.

In einer weiteren denkbaren Ausgestaltung der Erfindung weist der Sterilfilter in der Belüftungsleitung hydrophobe Eigenschaften auf.

Vorzugsweise ist weiterhin vorgesehen, dass in der Ablaufleitung ein weiterer Sterilfilter, bevorzugt stromabwärts der genannten Einmündung der Belüftungsleitung in die Ablaufleitung angeordnet ist.

Der oder die Sterilfilter in der Ablaufleitung weisen in bevorzugter Ausgestaltung der Erfindung hydrophile Eigenschaften auf.

Die Ablaufleitung und die Belüftungsleitung können einstückig ausgeführt sein, d.h. ein einziges Teil bilden oder auch mittels eines Konnektors miteinander verbindbar oder verbunden sein.

Die Ablaufleitung und/oder die Belüftungsleitung sind vorzugsweise als Schläuche ausgeführt.

In der Belüftungsleitung sind vorzugsweise Mittel zur Förderung von Luft durch die Belüftungsleitung in die Ablaufleitung angeordnet.

Dabei kann es sich um durch einen Nutzer anzutreibende Mittel oder um Mittel handeln, die eine Antriebseinheit aufweisen, wie z.B. ein Verdichter oder eine sonstige angetriebene Pumpenanordnung.

In einer vergleichsweise einfachen Ausführung können die Mittel als Blasebalg ausgeführt sein. Der Blasebalg kann einteilig mit der Belüftungsleitung ausgebildet sein und beispielsweise aus einem Folien- bzw. Schlauchmaterial ausgebildet sein.

Die Ablaufleitung und/oder die Belüftungsleitung kann einen Anschluss aufweisen, an dem ein Behältnis enthaltend ein Desinfektionsmittel angeordnet ist oder angeordnet werden kann. Somit ist es möglich, ein flüssiges oder gasförmiges Desinfektionsmittel in die Ablaufleitung einzuführen, um besonders sicher auszuschließen, dass sich in der Ablaufleitung Keime etc. ansiedeln, die im ungünstigsten Fall zum Patienten gelangen.

Die Ablaufleitung kann mit einem Konnektor zum Anschluss der Ablaufleitung an das Peritonealdialysegerät bzw. an den Patientenkatheter oder ein sonstiges Teil des Schlauchsystems versehen sein. Das genannte Absperrmittel kann sich innerhalb des Konnektors, aber auch außerhalb und getrennt von dem Konnektor befinden.

Denkbar ist es weiterhin, dass die Ablaufleitung mit einem Konnektor zum Anschluss der Ablaufleitung an das Peritonealdialysegerät, an den Patientenkatheter oder ein sonstiges Teil des Schlauchsystems versehen ist, wobei der Konnektor derart ausgeführt ist, dass dieser mit einer Seite an das Peritonealdialysegerät, an den Patientenkatheter oder ein sonstiges Teil des Schlauchsystems und mit der anderen Seite an einen bereits an der Ablaufleitung befindlichen Konnektor anschließbar oder angeschlossen ist.

In diesem Fall weist die Ablaufleitung eine Mehrzahl bereits benutzter Konnektoren auf, an die vor dem Ablassen der gebrauchten Dialyselösung ein neuer, steriler Konnektor angesetzt wird. Dieser neue Konnektor kann z.B. mit dem Patientenkatheter verbunden werden, der somit nur mit einem sterilen Bauteil, d.h. mit dem neu aufgesetzten Konnektor in Kontakt kommt.

Die genannten Absperrmittel können beispielsweise durch eine Klemme oder ein Ventil gebildet werden.

Denkbar ist es ferner, dass es sich bei dem Absperrmittel um ein Einwegeventil handelt.

Die vorliegende Erfindung betrifft des Weiteren ein Verfahren zur Belüftung einer Ablaufleitung eines Peritonealdialysegerätes nach einem der Ansprüche 1 bis 10, wobei das Absperrmittel geschlossen wird und wobei im Anschluss daran die Belüftung der Ablaufleitung mittels der Belüftungsleitung erfolgt, wodurch die gebrauchte Dialyselösung in einen Ablauf geleitet wird, bei dem es sich nicht um einen Beutel oder Behälter handelt.

Denkbar ist es, dass die Belüftung dadurch erfolgt, dass unter Druck Luft, vorzugsweise Umgebungsluft in die Ablaufleitung gefördert wird.

Die Förderung der Luft kann wie oben beschrieben manuell oder mittels eines Verdichters erfolgen.

Um die Ablaufleitung im Falle Ihrer Wiederverwendung steril zu halten, kann vorgesehen sein, dass vor, während oder nach der Belüftung der Ablaufleitung ein Sterilisationsmittel in die Ablaufleitung eingeleitet wird. Dabei besteht eine denkbare Variante darin, dass das Sterilisationsmittel der Luft beigemischt wird, die in die Ablaufleitung gefördert wird. Alternativ dazu wird das Sterilisationsmittel gesondert von der Luft zugegeben, die in die Ablaufleitung gelangt.

Ein Schlauchset zur Verwendung in einem Peritonealdialysegerät nach einem der Ansprüche 1 bis 10 und/oder in einem Verfahren nach einem der Ansprüche 11 bis 15 wird weiter beschrieben, aber nicht beansprucht, wobei das Schlauchset eine Ablaufleitung mit einem Konnektor zum Anschluss an das Peritonealdialysegerät, an den Patientenkatheter oder ein sonstiges Teil des Schlauchsystems und eine Belüftungsleitung aufweist, die an einer Einmündung mit der Ablaufleitung verbunden ist, so dass Luft aus der Belüftungsleitung in die Ablaufleitung eingeleitet werden kann, wobei Luftförderungsmittel vorgesehen sind, die über einen Konnektor an die Belüftungsleitung anschließbar ist oder einen Bestandteil der Belüftungsleitung bilden.

An dieser Stelle wird darauf hingewiesen, dass die Begriffe "ein" und "eine" nicht zwingend auf genau eines der Elemente verweisen, wenngleich dies eine mögliche Ausführung darstellt, sondern auch eine Mehrzahl der Elemente bezeichnen können. Ebenso schließt die Verwendung des Plurals auch das Vorhandensein des fraglichen Elementes in der Einzahl ein und umgekehrt umfasst der Singular auch mehrere der fraglichen Elemente.

Weitere Einzelheiten und Vorteile werden anhand eines in der Zeichnung dargestellten Ausführungsbeispiels näher beschrieben.

Es zeigen:
- Figur 1:: eine schematische Ansicht eines Schlauchsets mit einem angeschlossenen Dialysebeutel gemäß der Erfindung und
- Figur 2:: eine Ansicht eines Ablaufschlauchs mit miteinander verbindbaren Konnektoren.

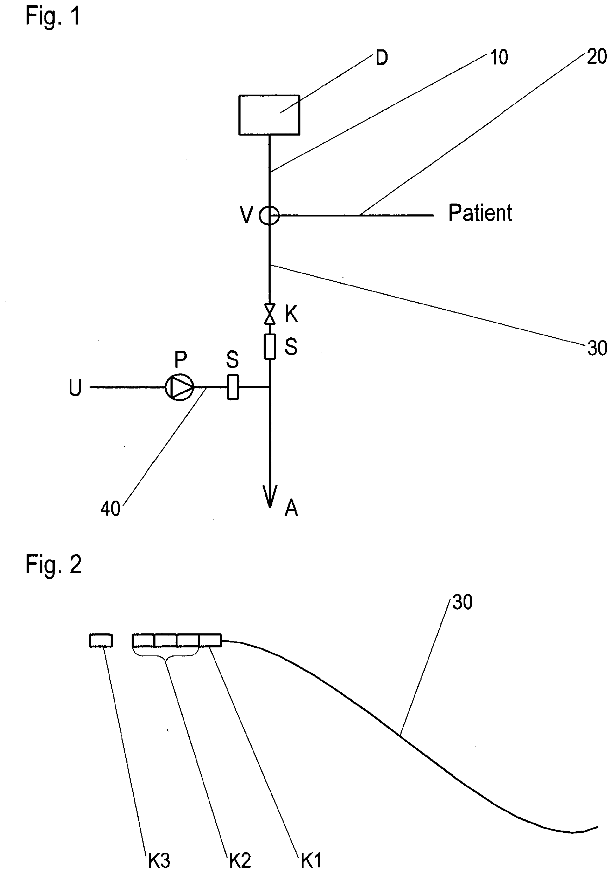

In Figur 1 ist mit dem Bezugszeichen D ein Beutel gekennzeichnet, der frische Dialyselösung enthält.

Während der Füllphase, wird der Verteiler V so gestellt, dass die Leitung 10 mit der Leitung 20 in Fluidverbindung steht, so dass frische Dialyselösung aus dem Beutel D in das Peritoneum des Patienten fließt.

Die Klemme K in der Ablaufleitung 30 ist in dieser Zeit geschlossen.

Nach Ablauf einer bestimmten Verweilzeit wird die gebrauchte Dialyselösung durch die Leitung 20 in die Ablaufleitung 30 geleitet, wozu der Verteiler V entsprechend verstellt wird, so dass eine Fluidverbindung zwischen den Leitungen 20, 30 besteht.

Die Klemme K wird dazu geöffnet und die gebrauchte Dialyselösung läuft zumindest teilweise in den Ablauf A, wie z.B. in ein Waschbecken, ein Duschbecken etc.

Da möglicherweise die höhendifferenzbedingte Druckdifferenz zwischen Patient und Ablauf A nicht ausreicht, um ein vollständiges Entleeren der Ablaufleitung 30 zu bewirken, mündet stromabwärts der Klemme K die Belüftungsleitung 40 in die Ablaufleitung. Diese steht an ihrem einen Ende mit der Ablaufleitung 30 in Verbindung und an ihrem anderen Ende mit der Umgebungsluft U, so dass Umgebungsluft U in die Belüftungsleitung 40 gesogen und von dieser in die Ablaufleitung 30 gefördert werden kann.

Um die vollständige Entleerung der Ablaufleitung 30 stromabwärts der Klemme K zu erreichen, wird die Klemme K geschlossen und der Verdichter, Blasbalg etc. in Betrieb genommen, wodurch Luft in die Ablaufleitung 30 stromabwärts der Klemme eingeführt wird und das darin noch verbliebende gebrauchte Dialysat vollständig in den Ablauf A geleitet wird.

Das Bezugszeichen S kennzeichnet einen Sterilfilter, der sicherstellt, dass aus der Umgebungsluft keine Keime in die Ablaufleitung 30 gelangen. Er befindet sich zwischen dem Verdichter etc. und der Einmündung der Leitung 40 in die Leitung 30.

Wie aus der Figur ersichtlich, befindet sich ein weiterer Sterilfilter S in der Ablaufleitung 30.

Die Ansteuerung bzw. der Betrieb der dargestellten Elemente, insbesondere des Verteilers V, der Klemme K und des Verdichters P können manuell oder gesteuert durch eine Steuerungseinheit und entsprechende Antriebsmittel erfolgen.

Figur 2 zeigt die Ablaufleitung 30, die an ihrem nicht zu dem Ablauf A gewandten Ende mit einem Konnektor versehen ist, der z.B. an den Verteiler oder an den nicht dargestellten Patientenkatheter anschließbar ist. Der Patientenkatheter ist ein in das Peritoneum führender Schlauchabschnitt, der fest mit dem Patienten verbunden ist und der über einen Konnektor mit einem Schlauchset verbunden wird, um Dialyselösung in das Peritoneum einführen und aus diesem abführen zu können.

Die Leitung 20 gemäß Figur 1 kann beispielsweise aus einem ersten Abschnitt bestehen, der durch den Patientenkatheter gebildet wird und aus einem zweiten Abschnitt, der zu dem Verteilter V führt, was in Figur 1 nicht dargestellt ist.

Der Konnektor K1 ist der ursprünglich an der Ablaufleitung 30 befindliche Konnektor. Die Konnektoren K2 sind nacheinander aufgesteckt worden und zwar pro Behandlung je ein frischer, steriler Konnektor K2. Der Konnektor K3 ist ein neuer, steriler Konnektor, der für den nächsten Ablauf der Dialyselösung aus dem Peritoneum des Patienten verwendet wird. Dieser wird auf den letzten der bereits vorhanden Konnektoren K2 aufgesteckt, so dass der Patientenkatheter oder ein anderes Konnektorelement, das mit dem Konnektor K3 verbunden wird, steril bleibt.

Die Konnektoren können eine geschlitzte Membran aufweisen, die im konnektierten Zustand geöffnet wird und somit einen Fluiddurchgang freigibt.

Die Verbindung der Konnektoren kann durch ein Gewinde, eine Rastverbindung etc. erfolgen.

## Patentansprüche

1. Peritonealdialysegerät mit Mitteln zur Durchführung einer Peritonealdialysebehandlung, wobei das Gerät eine Ablaufleitung (30) aufweist, die mit dem Patientenkatheter in Verbindung steht oder verbindbar ist und durch die gebrauchte Dialyselösung in einen Ablauf geleitet wird, **wobel** die Ablaufleitung (30) nicht in einen Drainbehälter mündet und dass die Ablaufleitung (30) an einer Einmündung mit einer Belüftungsleitung (40) verbunden ist, mittels derer die Belüftungsleitung (40) belüftbar ist, wobei in der Ablaufleitung (30) stromaufwärts der Einmündung und/oder in der Belüftungsleitung (40) ein Sterilfilter (S) angeordnet ist und wobei in der Ablaufleitung (30) stromaufwärts der Einmündung ein Absperrmittel (K) vorgesehen ist, mittels dessen die Ablaufleitung (30) zeitweise absperrbar ist, **dadurch gekennzeichnet, dass** die Länge der Ablaufleitung (30) mindestens 2 meter beträgt.

2. Peritonealdialysegerät nach Anspruch 1, **dadurch gekennzeichnet, dass** es sich bei dem Peritonealdialysegerät um ein CAPD-Set handelt.

3. Peritonealdialysegerät nach einem der vorhergehenden Ansprüche, **dadurch gekennzeichnet, dass** in der Ablaufleitung (30) ein weiterer Sterilfilter (S) stromabwärts der Einmündung vorgesehen ist.

4. Peritonealdialysegerät nach einem der vorhergehenden Ansprüche, **dadurch gekennzeichnet, dass** in der Belüftungsleitung (40) Mittel zur Förderung von Luft durch die Belüftungsleitung (40) in die Ablaufleitung (30) angeordnet sind.

5. Peritonealdialysegerät nach Anspruch 4, **dadurch gekennzeichnet, dass** es sich bei den Mitteln um durch einen Nutzer anzutreibende Mittel oder um Mittel handelt, die eine Antriebseinheit aufweisen.

6. Peritonealdialysegerät nach Anspruch 4 oder 5, **dadurch gekennzeichnet, dass** die Mittel als Blasebalg oder als Verdichter (P) ausgebildet sind.

7. Peritonealdialysegerät nach einem der vorhergehenden Ansprüche, **dadurch gekennzeichnet, dass** die Ablaufleitung (30) und/oder die Belüftungsleitung (40) einen Anschluss aufweist, an dem ein Behältnis enthaltend ein Desinfektionsmittel angeordnet ist oder angeordnet werden kann.

8. Peritonealdialysegerät nach einem der vorhergehenden Ansprüche, **dadurch gekennzeichnet, dass** die Ablaufleitung (30) mit einem Konnektor zum Anschluss der Ablaufleitung (30) an das Peritonealdialysegerät versehen ist und dass sich das Absperrmittel in dem Konnektor angeordnet ist.

9. Peritonealdialysegerät nach einem der vorhergehenden Ansprüche, **dadurch gekennzeichnet, dass** die Ablaufleitung (30) mit einem Konnektor zum Anschluss der Ablaufleitung an das Peritonealdialysegerät versehen ist und dass das der Konnektor derart ausgeführt ist, dass mit einer Seite an das Peritonealdialysegerät und mit der anderen Seite an einen bereits an der Ablaufleitung (30) befindlichen Konnektor versehen ist.

10. Peritonealdialysegerät nach einem der vorhergehenden Ansprüche, **dadurch gekennzeichnet, dass** es sich bei dem Absperrmittel um ein Einwegeventil handelt.

11. Verfahren zur Belüftung einer Ablaufleitung eines Peritonealdialysegerätes nach einem der Ansprüche 1 bis 10, **dadurch gekennzeichnet, dass** zunächst das Absperrmittel (K) geschlossen wird und dass im Anschluss daran die Belüftung der Ablaufleitung (30) mittels der Belüftungsleitung (40) erfolgt, wodurch die gebrauchte Dialyselösung in einen Ablauf geleitet wird, bei es sich nicht um einen Drainbehälter handelt.

12. Verfahren nach Anspruch 11, **dadurch gekennzeichnet, dass** die Belüftung dadurch erfolgt, dass unter Druck Luft, vorzugsweise Umgebungsluft in die Ablaufleitung (30) gefördert wird.

13. Verfahren nach Anspruch 12, **dadurch gekennzeichnet, dass** die Förderung manuell oder mittels eines Verdichters (P) erfolgt.

14. Verfahren nach einem der Ansprüche 11 bis 13, **dadurch gekennzeichnet, dass** vor, während oder nach der Belüftung der Ablaufleitung (30) ein Sterilisationsmittel in die Ablaufleitung (30) eingeleitet wird.

15. Verfahren nach Anspruch 14, **dadurch gekennzeichnet, dass** das Sterilisationsmittel der Luft beigemischt wird, die in die Ablaufleitung (30) gefördert wird oder dass Sterilisationsmittel gesondert von der Luft zugegeben wird, die in die Ablaufleitung (30) gelangt.

## Claims

1. A peritoneal dialysis machine having means for carrying out a peritoneal dialysis treatment, wherein the machine has a drain line (30) which is connected or connectable to the patient catheter and through which consumed dialysis solution is conducted into a drain, wherein the drain line (30) does not open into a drainage container, and in that the drain line (30) is connected at an opening to a venting line (40) by means of which the venting line (40) can be vented, wherein a sterile filter (S) is arranged in the drain line (30) upstream of the opening and/or in the venting line (40), and wherein a cut-off means (K) by means of which the drain line (30) can be cut off at times is provided in the drain line (30) upstream of the opening, **characterised in that** the length of the drain line (30) is at least 2 metres.

2. The peritoneal dialysis machine according to claim 1, **characterised in that** the peritoneal dialysis machine is a CAPD set.

3. The peritoneal dialysis machine according to any one of the preceding claims, **characterised in that** a further sterile filter (S) is provided in the drain line (30) downstream of the opening.

4. The peritoneal dialysis machine according to any one of the preceding claims, **characterised in that** means for conveying air through the venting line (40) into the drain line (30) are arranged in the venting line (40).

5. The peritoneal dialysis machine according to claim 4, **characterised in that** the means are means to be actuated by a user or means that have a drive unit.

6. The peritoneal dialysis machine according to claim 4 or 5, **characterised in that** the means are configured as bellows or as a compressor (P).

7. The peritoneal dialysis machine according to any one of the preceding claims, **characterised in that** the drain line (30) and/or the venting line (40) has a connection at which a container containing a disinfectant is arranged or can be arranged.

8. The peritoneal dialysis machine according to any one of the preceding claims, **characterised in that** the drain line (30) is provided with a connector for connecting the drain line (30) to the peritoneal dialysis machine, and **in that** the cut-off means is arranged in the connector.

9. The peritoneal dialysis machine according to any one of the preceding claims, **characterised in that** the drain line (30) is provided with a connector for connecting the drain line to the peritoneal dialysis machine, and **in that** the connector is configured such that it is provided at the peritoneal dialysis machine at one side and at a connector already located at the drain line (30) at the other side.

10. The peritoneal dialysis machine according to any one of the preceding claims, **characterised in that** the cut-off means is a one-way valve.

11. A method for venting a drain line of a peritoneal dialysis machine according to any one of claims 1 to 10, **characterised in that** the cut-off means (K) is closed first, and **in that** the drain line (30) is vented subsequent thereto by means of the venting line (40), whereby the consumed dialysis solution is conducted into a drain which is not a drain container.

12. The method according to claim 11, **characterised in that** the venting takes place by conveying air, preferably ambient air, into the drain line (30) at pressure.

13. The method according to claim 12, **characterised in that** the conveying is effected manually or by means of a compressor (P).

14. The method according to any one of claims 11 to 13, **characterised in that** a sterilising agent is introduced into the drain line (30) before, during, or after the venting of the drain line (30).

15. The method according to claim 14, **characterised in that** the sterilizing agent is admixed to the air that is conveyed into the drain line (30), or **in that** the sterilising agent is added separately from the air that enters into the drain line (30).

## Revendications

1. Appareil de dialyse péritonéale doté de moyens pour exécuter un traitement de dialyse péritonéale, l'appareil présentant une conduite d'évacuation (30) qui est ou peut être reliée au cathéter du patient et par laquelle de la solution de dialyse usagée est guidée dans une évacuation, la conduite d'évacuation (30) ne débouchant pas dans un contenant de drainage, et en ce que la conduite d'évacuation (30) est reliée, au niveau d'une embouchure, à une conduite de ventilation (40), au moyen de laquelle la conduite de ventilation (40) peut être ventilée, un filtre stérile (S) étant disposé dans la conduite d'évacuation (30) en amont de l'embouchure et/ou dans la conduite de ventilation (40), et un moyen de blocage (K) étant prévu dans la conduite d'évacuation (30) en amont de l'embouchure, au moyen duquel la conduite d'évacuation (30) peut être temporairement bloquée, **caractérisé en ce que** la longueur de la conduite d'évacuation (30) est d'au moins 2 mètres.

2. Appareil de dialyse péritonéale selon la revendication 1, **caractérisé en ce que** l'appareil de dialyse péritonéale est un set de DPCA.

3. Appareil de dialyse péritonéale selon l'une des revendications précédentes, **caractérisé en ce qu'**un autre filtre stérile (S) est prévu dans la conduite d'évacuation (30) en aval de l'embouchure.

4. Appareil de dialyse péritonéale selon l'une des revendications précédentes, **caractérisé en ce que** des moyens destinés à transporter de l'air par la conduite de ventilation (40) jusqu'à la conduite d'évacuation (30) sont disposés dans la conduite de ventilation (40).

5. Appareil de dialyse péritonéale selon la revendication 4, **caractérisé en ce que** les moyens sont des moyens destinés à être entraînés par un utilisateur ou des moyens qui présentent une unité d'entraînement.

6. Appareil de dialyse péritonéale selon la revendication 4 ou 5, **caractérisé en ce que** les moyens sont réalisés sous la forme d'un soufflet ou d'un compresseur (P).

7. Appareil de dialyse péritonéale selon l'une des revendications précédentes, **caractérisé en ce que** la conduite d'évacuation (30) et/ou la conduite de ventilation (40) présente un raccord, sur lequel est ou peut être disposé un récipient contenant un agent désinfectant.

8. Appareil de dialyse péritonéale selon l'une des revendications précédentes, **caractérisé en ce que** la conduite d'évacuation (30) est pourvue d'un connecteur pour le raccordement de la conduite d'évacuation (30) à l'appareil de dialyse péritonéale et **en ce que** le moyen de blocage est disposé dans le connecteur.

9. Appareil de dialyse péritonéale selon l'une des revendications précédentes, **caractérisé en ce que** la conduite d'évacuation (30) est pourvue d'un connecteur pour le raccordement de la conduite d'évacuation à l'appareil de dialyse péritonéale et **en ce que** le connecteur est conçu de telle manière qu'il peut être relié par un côté à l'appareil de dialyse péritonéale et par l'autre côté à un connecteur se trouvant déjà sur la conduite d'évacuation (30).

10. Appareil de dialyse péritonéale selon l'une des revendications précédentes, **caractérisé en ce que** le moyen de blocage est une valve antiretour.

11. Procédé de ventilation d'une conduite d'évacuation d'un appareil de dialyse péritonéale selon l'une des revendications 1 à 10, **caractérisé en ce que** le moyen de blocage (K) est d'abord fermé et **en ce que**, ensuite, la ventilation de la conduite d'évacuation (30) est effectuée au moyen de la conduite de ventilation (40), moyennant quoi la solution de dialyse usagée est guidée dans une évacuation, celle-ci n'étant pas un contenant de drainage.

12. Procédé selon la revendication 11, **caractérisé en ce que** la ventilation est effectuée par le fait que de l'air, de préférence de l'air ambiant, est transporté sous pression dans la conduite d'évacuation (30).

13. Procédé selon la revendication 12, **caractérisé en ce que** le transport s'effectue manuellement ou au moyen d'un compresseur (P).

14. Procédé selon l'une des revendications 11 à 13, **caractérisé en ce qu'**un agent stérilisant est introduit dans la conduite d'évacuation (30) avant, pendant ou après la ventilation de la conduite d'évacuation (30).

15. Procédé selon la revendication 14, **caractérisé en ce que** l'agent stérilisant est mélangé à l'air qui est transporté dans la conduite d'évacuation (30) ou **en ce que** de l'agent stérilisant est ajouté séparément de l'air qui parvient dans la conduite d'évacuation (30).
